# EUROPEAN PATENT SPECIFICATION

(11) **EP 3 035 898 B1**
(45) Date of publication and mention of the grant of the patent: **15.09.2021**
(21) Application number: 14838260.9
(22) Date of filing: 22.08.2014
(51) Int. Cl.: A61F 13/12, A61F 13/02, A61F 9/04

(54) **IMPROVED EYE PATCH**
VERBESSERTES AUGENPFLASTER
PANSEMENT OCULAIRE AMÉLIORÉ

(30) Priority: 23.08.2013 AU 2013903209
(43) Date of publication of application: 29.06.2016
(73) Proprietor: Innovgas Pty Ltd, Launceston, Tasmania 7250 (AU)
(72) Inventor: WALLIS, Andrew, Launceston, Tasmania 7250 (AU)
(74) Representative: O'Connor, Dominic David
(86) International application number: PCT/AU2014/050194
(87) International publication number: WO 2015/024076

(56) References cited:
- US-A- 3 092 103
- US-A- 4 867 146
- US-A- 5 127 423
- US-A- 5 887 590
- US-A1- 2002 100 481
- None

## Description

### FIELD OF THE INVENTION

The present invention relates to a perioperative eye protection patch for retaining a patient's eye closed during a medical procedure and in one aspect relates to an eye patch having different adhesive portions that retain the eyelids closed during the medical procedure, while inhibiting injury to the patient's eyelids when the eye patch is removed.

It is envisaged that the eye patch will be used on an anaesthetised or sedated patient during a medical procedure, however the eye patch may also be used on an unconscious patient in an emergency medical situation. The terms rigidity or stiffness used throughout the specification and claims describes the relative stiffness of the different parts of the eye patch. The term is not to be understood as meaning rigid or stiff, in that the part is inflexible, but rather describes differing flexibility of the parts of the eye patch, wherein one part has less flexibility to provide a support for another part of the eye patch

### BACKGROUND OF THE INVENTION

During a medical procedure the eyelids of the anaesthetised or sedated patient may open. The complete or partial opening of the eyelids can lead to injury or irritation of the patient's eye by direct trauma or corneal drying leading to ulceration.

Corneal abrasion is a common eye injury, which is caused by clothing or other items coming into contact with the patient's eye during surgery. Furthermore, injury or irritation may result from fluids and creams used during the medical procedure, such skin preparations, coming into contact with the eye between the parted eyelids of the patient.

It is known that tear production of an anaesthetised or sedated patient is significantly reduced during genera! anaesthesis, which accelerates the drying out of the eye and potentially ulceration. Furthermore, the usual random motion of the eye during sleep is decreased during sedation and anaesthesia. Accordingly, the open eye may quickly dry out leading to injury or irritation, including blurred vision, conjunctivitis and potentially permanent degradation of the optical performance of the cornea.

Often a strip of adhesive surgical tape is applied across the upper and lower eyelids to maintain them in a closed configuration during surgery. Some medical tapes have an adhesive layer that is relatively weak which means that the tape can be easily displaced by the medical personnel during the medical procedure, or the tape can become dislodged due to increased moisture on the skin or due to the fluids used during the medical procedure. Other medical tapes, such as gauze tapes, have an adhesive layer that binds strongly to the patient's shin and eyelashes. When such tape is removed the patient's eyelashes may be plucked and the skin of the eyelids, especially in older patients, may be torn or bruised, thereby causing increased discomfort to the patient.

Various opaque eye patches have been developed that cover the eyelids of the patient, either partially or completely. An anaesthetist is not however able to view the eyelids to ensure they are closed. To overcome this problem patches, such as that disclosed in United States Patent 5,887,590 to PRICE, have been proposed. The patch disclosed in PRICE comprises a flexible transparent backing material having a rear surface substantially covered by an adhesive coating. The patch further includes a graspable, adhesive-free, portion that forms a tab. Although the eye can be viewed through this type of patch the patient's eyelashes and edges of the eyelids adhere strongly to the adhesive coating, which causes problems when it is being removed, as previously discussed.

Another patch disclosed in the prior art, is found in WO00/02507 to ABBASI that teaches an eye shield for retaining the eyelids of an anesthetised patient closed during a surgical procedure, to protect the eye from injury. The rear surface of the eye shield of ABBASI includes upper and lower adhesive portions for attachment to respective upper and lower eyelids of the patient, at a distance from the patient's eyelashes. A non-adhesive transparent window allows the anaesthetist to view the patient's eye therethrough during surgery. The transparent window corresponds to the position of the closed eyelids and prevents adhesion of the patch to the eyelashes and edges of the eyelids. However one of the problems with this type of patch is that the patient's eyelids can still open, which can lead to drying out of the eye.

It should be appreciated that any discussion of the prior art throughout the specification is included solely for the purpose of providing a context for the present invention and should in no way be considered as an admission that such prior art was widely known or formed part of the common general knowledge in the field as ft existed before the priority date of the application.

The phrase 'bond strength', or similar, used throughout the specification generally describes the properties of the adhesive and how well it adheres to the substrate (skin or eyelashes), and how long it is able to maintain its adhesive properties. The phrase 'bond strength' also incorporates the concepts of peel strength and tack strength. Accordingly, the terms adhesion or adhesive bond strength used throughout the specification relates generally to the strength of the adhesive or adhesives used.

### SUMMARY OF THE INVENTION

The invention provides an eye patch as claimed in claim 1.

It should be appreciated that the higher the adhesive bond strength, the more force is required to peel one object from the other. Accordingly, the adhesive bond strength or tack between the eye patch and the face of the patient is different for the transparent second portion compared to the first portion of the eye patch. The first portion of the eye patch is preferably affixed to the patient's face adjacent the edge of the patent's eye socket or bony orbital rim of the eye, around the brow and cheekbone, and at either side of the eye adjacent the nose and temple.

The backing is in use attached to the face of the patient, surrounding and at a distance from the eyelashes. The backing may extend over an outer part of said upper and lower eyelids provided it does not extend over the edge of the eyelids and eyelashes.

In one form the second portion is a lightweight, flexible film or membrane. Such films or membranes would usually be impractical to handle, however since it is attached to the circumferential first portion and extends over the central opening, a very thin film or membrane can be used for application to the eyelids of the patient. Therefore the relative rigidity of the first portion enables handling of a very light, flexible second portion that has characteristics which are beneficial to the comfort of the patient, and further inhibits injury to the patient's face when the eye patch is removed.

The tab may be spaced apart from the transparent film and is preferably on an opposite side of the eye patch to said transparent film.

The backing could be understood to comprise an adhesive bandage portion of a material used for medical purposes, such as an open weave gauze material having an adhesive layer, or a conventional medical tape material or sticking plaster.

In one form the backing has a first adhesive coating in the form of a pressure-sensitive adhesive rear surface, similar to medical tapes used for surgical or medical purposes. The first adhesive coating may be a hypoallergenic adhesive that is designed to hold firmly onto the skin of said patient.

The central opening of the backing and the transparent film form a transparent viewing window that permits an anaesthetist or other medical practitioner to be able to see the eyelids to ensure they are closed throughout the medical procedure. The second adhesive coating of the transparent film inhibits the opening of the eyelids. The second adhesive coating is strong enough to prevent the movement of the eyelids, however is weak enough that it can be removed from the face of the patient without binding to the eyelashes and edges of the eyelids, which would result in the plucking of the eyelashes, or tearing of the skin around the edges of the eyelids, especially in older patients.

As will now be appreciated the first and second adhesive coatings are for attachment to different regions of the patient's face, which means that the eye patch inhibits plucking of eyelashes and damage to the skin along the edges of the upper and lower eyelids adjacent the eyelashes during removal of the eye patch. Furthermore, the backing with its adhesive coating inhibits the eye patch from being dislodged during the medical procedure, and the transparent film with its adhesive coating having weaker adhesive bond strength, inhibits the eyelid from opening during the medical procedure while inhibiting damage when the patch is removed.

Preferably the eye patch is a single use sterile dressing, one for each eye, that keeps the eyelids closed during surgery, thereby inhibiting corneal drying, corneal scarring and other eye damage.

Preferably the backing is thicker than the transparent film. The backing may be between 2-10 times the thickness of the transparent film, or any other ratio to provide a backing that has a degree of rigidity while providing for a flexible, thin transparent film for overlaying the eyelids of the patient. In one form the transparent film has a thickness of 0.5mm and the backing has a thickness of 2mm. The thicker backing is opaque and provides a degree of rigidity to assist when applying the eye patch to the patient's face.

The transparent film is relatively thin and flexible in comparison to the backing. Accordingly, the backing has greater rigidity or stiffness compared to the transparent film. This facilitates handling and application of the eye patch by the practitioner and also provides a relatively rigid backing for holding the eye patch in place during the medical procedure. The eye patch is therefore still flexible enough to be conformed to the contours of the patient's face but is rigid enough that it is not difficult to handle.

In one form the use of a thicker material for the backing, compared to the transparent film, may be used to increase the rigidity of the eye patch. Alternatively, the backing may be made from a different type of material to the transparent film, include ridges, or have a composite construction, to thereby increase rigidity of the backing while still permitting the eye patch to conform to the face of the patient.

In one form the backing may be printed onto the transparent film and the first and second adhesive coating can then be applied.

The eye patch may also have a visual delineation between the edge of the central opening of the backing and the transparent viewing window. This facilitates correct placement of the eye patch relative to the eyelids. In one form, the backing may be constructed from an opaque material that would provide the visual delineation between the different parts of the eye patch. In another form, the backing may be constructed from a transparent material that is provided with alignment markings, such as an outline or shading, to ensure the central viewing window is positioned correctly with respect to the eye and eyelashes. The alignment markings may be provided by way of printing the upper surface of the backing, however it should be appreciated that other ways of providing alignment markings could also be used.

The eye patch, and in particular the transparent film, is preferably fluid resistant or repellent to inhibit skin preparations or fluids/oils, used during the medical procedure, from penetrating the eye patch and coming into contact with the eye. The eye patch therefore acts to protect the eye from contact with chemicals that may affect the eye of the patient.

Furthermore, the eye patch is configured to conform to the face or outer surface of eye socket and eye, wherein the backing is attached to the skin circumferentially around the eye adjacent the edge of the eye socket. This ensures that the eye is sealed off from the surrounding environment, thereby preventing fluids and creams flowing over the surface of the skin and in between the closed eyelids, which could result in irritation or injury. The eye patch therefore inhibits chemical passing through the patch, or under the edge of the patch, and into the eye.

In one form the tab having an outer non-adhesive surface extends outwardly from, or covers a portion of, said backing. Preferably the eye patch has two non-peelable tabs, one at each side of the backing, each having a respective non-adhesive outer surface. Accordingly a tab may be positioned at each side of the eye patch for positioning adjacent the nose and temple to aid in the application and removal of the eye patch.

In this way the eye patch can be removed from the medial or lateral edge of the eye socket. As the eye patch is removed in a direction parallel to the edge of the eyelids the chance of the eyelids being pulled apart is reduced. Furthermore, the direction of removal of the eye patch further inhibits damage because the transparent film is gradually removed along the length of the eyelid edge rather than impinging along the whole width of the eyelid at the same time.

In one form the eye patch is constructed from three layers, an upper layer comprising the transparent film having an underside substantially covered by said second adhesive coating, a middle layer comprising the backing having the central opening extending therethrough and an underside substantially covered by the first adhesive coating, and a lower layer comprising two spaced apart tabs having non-adhesive underside surfaces, the upper and middle layers having similar outer dimensions. In one form the upper layer is bonded to and completely covers the middle layer and the tabs are bonded to and cover underside portions at opposite sides of the middle layer. In another form the transparent film is attached to the backing around the edge of the central opening wherein the transparent film is spaced apart from the tabs.

The underside of both the backing and transparent film are preferably covered by a peelable protective sheet, which protects the first and second adhesive coatings prior to use.

The transparent film in one form is 30µm polyurethane film. The backing in one form is 50gsm non-woven extensible material. The tabs in one form are constructed from 65gsm paper or olefin fibre, and include printing to indicate the tab. The peelable protective sheet in one form comprises 65gsm silicon release paper.

The first adhesive coating in one form is a pressure sensitive acrylic adhesive, such as 30gsm AROSET® 951005. The second adhesive coating in one form is a pressure sensitive acrylic adhesive, such as 25gsm SOLUCRYL® 147.

The width of the eye patch is between 60-90mm and is preferably 83mm and the height of the eye patch is between 25-45mm and in preferably 31mm.

### BRIEF DESCRIPTION OF THE DRAWINGS

The accompanying drawings, which are incorporated in and constitute a part of this specification, illustrate an implementation of the invention and, together with the description and claims, serve to explain the advantages and principles of the invention. In the drawings,
- Figure 1: is a front view of an improved eye patch of the present invention affixed to a patient's face;
- Figure 2: is a front view of the eye patch of figure 1;
- Figure 3: is a cross-sectional perspective view through A-A of the eye patch of figure 2;
- Figure 4: is an exploded view of a second embodiment of the eye patch of the present invention, illustrating the upper transparent film, middle layer and the tab forming lower layer and the different thickness of the upper transparent film and middle layer;
- Figure 5: is a perspective view of the eye patch of figure 4;
- Figure 6: is a perspective view of the eye patch of figure 5 with a peelable protective sheet;
- Figure 7: is an enlarged view of a patient's face illustrating the attachment of the eye patch; and
- Figure 8: is an exploded view of a third embodiment of the eye patch.

### DETAILED DESCRIPTION OF THE ILLUSTRATED AND EXEMPLIFIED EMBODIMENTS

Similar reference characters indicate corresponding parts throughout the drawings. Dimensions of certain parts shown in the drawings may have been modified and/or exaggerated for the purposes of clarity or illustration.

Referring to the drawings for a more detailed description, an improved eye patch 10 is illustrated, demonstrating by way of examples, arrangements in which the principles of the present invention may be employed. The figures illustrate an eye patch 10 that is used to retain eyelids 21, 22 of eye 12 of the patient 14, closed during a medical procedure. The eye patch 10 includes a transparent viewing window 16 to enable observation of the eye 12 therethrough during the procedure.

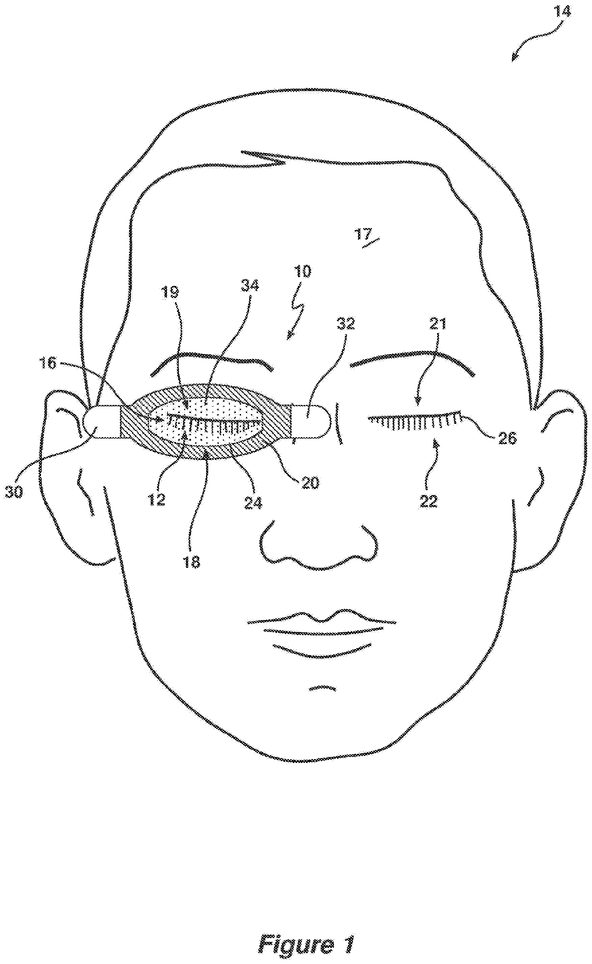

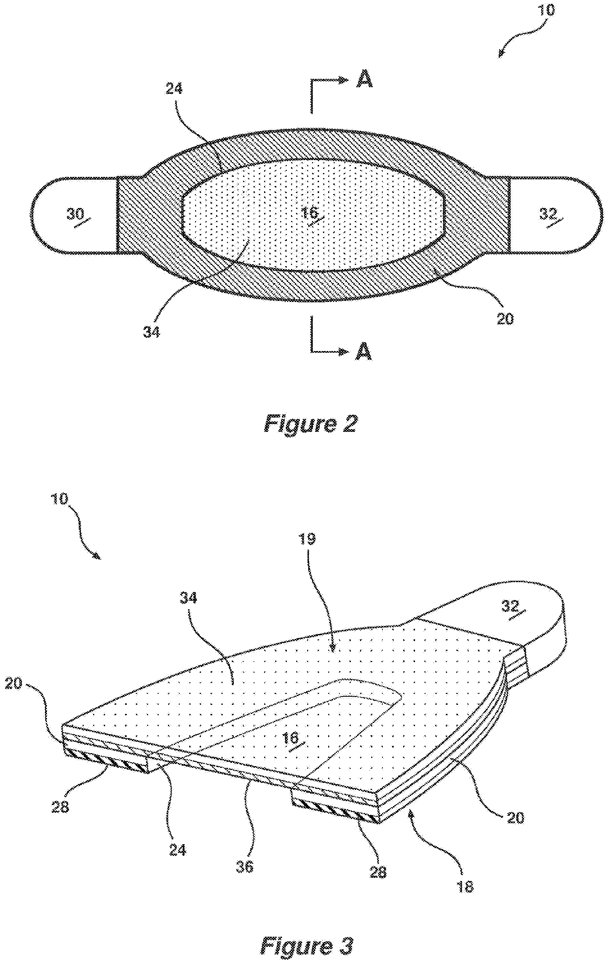

The eye patch 10 is releasably attached or mounted to the face 17 of the patient 14 to retain the patient's eyelids 21, 22 closed. As illustrated in figure 3, the eye patch 10 includes a first portion 18 having a first adhesive coating 28 for securing the eye patch 10 to the face 17 of the patient 14, as shown in figure 1. The first portion 18 having a central opening 24 extending therethrough. A second portion 19 extends over the central opening 24 and has a second adhesive coating 36, as illustrated in figure 3. Importantly, the adhesive bond strength of the second adhesive coating 36 is weaker than the adhesive bond strength of the first adhesive coating 28, to thereby retain the eye patch 10 in position during the medical procedure to inhibit the eyelids 21, 22 from opening, while inhibiting injury to the patient's eyelids 21, 22 or plucking of the eyelashes 26, when the eye patch 10 is removed at the end of the medical procedure.

In one embodiment as illustrated in figures 1 to 3, the eye patch 10 includes a first portion 18 comprising a backing 20 dimensioned to extend substantially over said patient's upper 21 and lower 22 eyelids, the backing 20 having the central opening 24 extending therethrough, so that it can be positioned to correspond to the location of the patient's eyelashes 26 when the eye 12 is closed. As illustrated in figure 3 the first adhesive coating 28 extends over an underside of the backing 20. The first adhesive coating 28 is used to secure the backing 20 circumferentially around the outer edge of the patent's eye 12, at a distance from said eyelashes 26, as illustrated in figure 1. The backing 20 may extend over and be attached to an outer part of the patient's upper and lower eyelids 21, 22 but does not extend over the edge of the upper and lower eyelids 21, 22 adjacent the eyelashes 26.

Tabs 30, 32 are positioned at opposite side of the eye patch 10 to correspond to the medial or lateral edges of the eye 12. The tabs 30, 32 have a non-adhesive surface to enable a practitioner to grasp the eye patch 10 without it adhering to their fingers or gloves. The tabs 30, 32 assist in the attachment and removal of the eye patch 10.

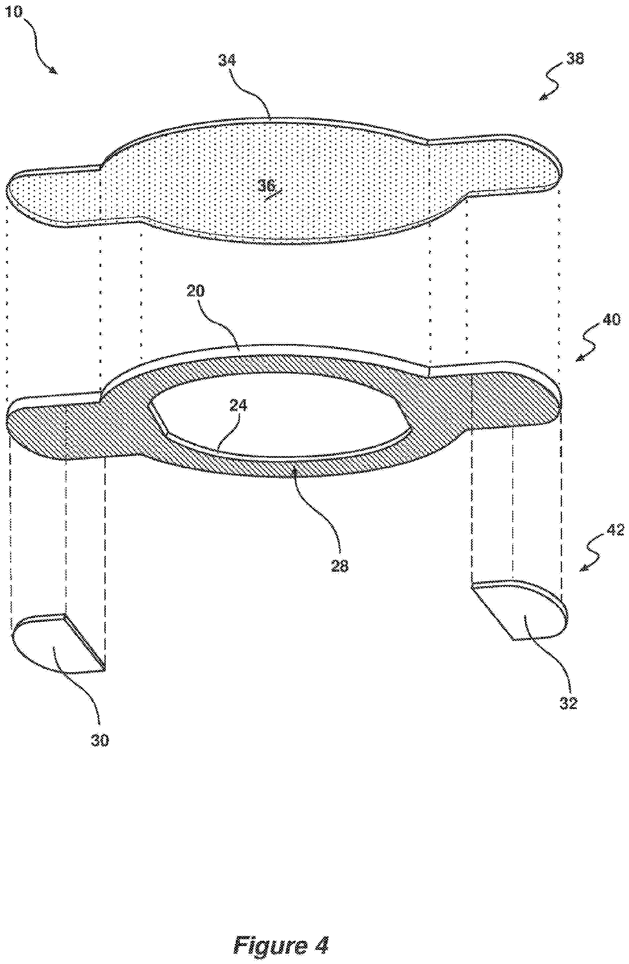

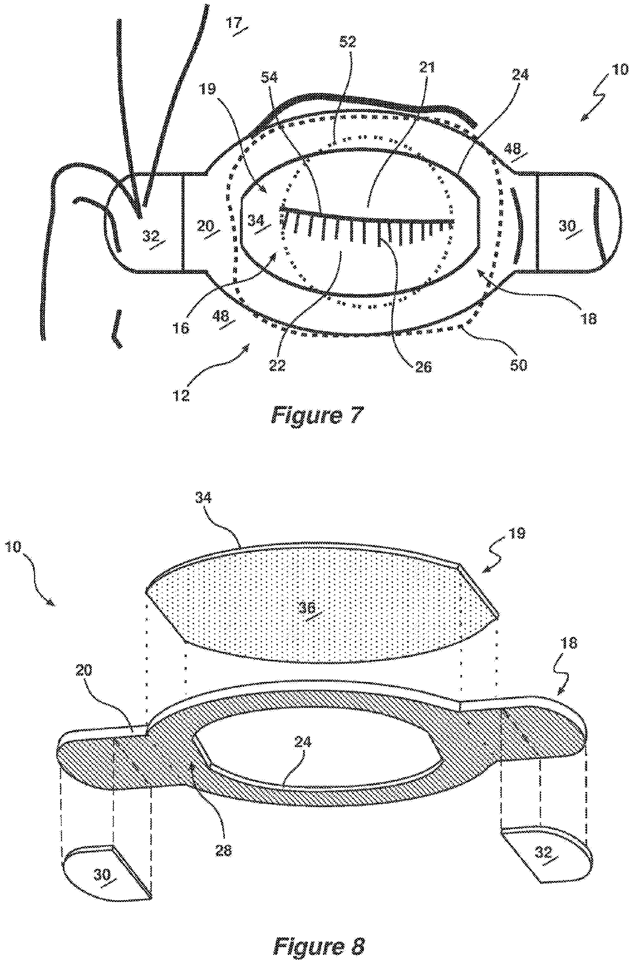

It should be appreciated that the tabs 30, 32 may extend outwardly from sides of the eye patch, as illustrated in the first embodiment of figure 3, or the tabs 30, 32, may be formed by covering a part of the backing, as illustrated in the second embodiment of figures 4 to 6. Furthermore the tabs 30, 32 may be spaced apart laterally from the second portion 19, as illustrated in figure 8.

The second portion 19 comprising a transparent film 34 that extends over the central opening 24 to enable viewing of the patient's eye 12 therethrough. The transparent film 34 has the second adhesive coating 36 for inhibiting opening of the eye 12 during the medical procedure. The reader would now appreciate that the adhesion or bond strength of the second adhesive coating 36 is weaker than the adhesion or bond strength of the first adhesive coating 28. The weaker bond to the eyelashes 36 and the area immediately adjacent the edges of the upper and lower eyelids 21, 22, inhibits injury to the patient when the eye patch 10 is removed. This is particularly important for older patients, whose skin is thin and can be easily torn or bruised causing discomfort.

The higher the adhesive bond strength, the higher will be the number of Newtons required to peel one object from another object. The difference in the bond strength for the different portions 18, 19 of the eye patch inhibits injury, such as plucking of eyelashes or tearing of the skin along the edges of the eyelids, while still ensuring that the patch does not become dislodged during use.

In a second embodiment as illustrated in figures 4 to 6 the eye patch 10 is constructed from three layers, an upper layer 38 comprising the transparent film 34 having an underside covered by the second adhesive coating 36, a middle layer 40 comprising the backing 20 having an underside covered by the first adhesive coating 28 and a lower layer 42 comprising the two tabs 30, 32 having non-adhesive underside surfaces.

As can be seen from the exploded view in figure 4 the upper and middle layers 38, 40 have similar outer dimensions, with the upper layer extending over the central opening 24. The underside of the transparent film 34 is substantially covered by coating 36, which means that during manufacture the upper layer 38 can be bonded to and completely cover the upper surface of the middle layer 40. Likewise the tabs can be simple disc-shaped or dome-shaped pieces of paper that are affixed to the underside of the middle layer 38 that is covered by adhesive coating 28.

As illustrated in figure 4 to 6 the backing 20 is thicker than the transparent film 34. The thicker backing 20 provides a degree of rigidity or stiffness to assist the practitioner when handling and applying the eye patch 10 to the patient's face 17. Furthermore, the rigidity or stiffness of the backing 20 assists in holding the eye patch 10 in place during the medical procedure.

The reader should however appreciate that the backing 20 may be made from a different material to the transparent film 34, include ridges, or have a composite construction, to thereby increase rigidity or stiffness of the backing. It should also be appreciated that the difference between the respective thicknesses of the backing 20 and transparent film 34 may be greater than illustrated in the figures. The transparent film 34 may be very thin in comparison to the backing 20 and the figures simply illustrate, for the purposes of discussion, a possible thickness of the transparent film 34.

As illustrated in the figures the eye patch 10 has a visual delineation between the edge of the central opening 24 and the transparent viewing window 16. This facilitates correct placement of the eye patch relative to the eye 12 and eyelashes 26.

In the present embodiments the backing 20 is constructed from an opaque material to thereby provide the visual delineation between the different parts of the eye patch 10. However, the reader should appreciate that the backing 20 may be constructed from a transparent material, which is provided with alignment markings, such as an outline of the eye or shading, to ensure the central viewing window is positioned correctly with respect to the eye.

In the present embodiments the eye patch 10 has two non-peelable tabs at opposite ends of the backing 20, each having a respective non-adhesive outer surface. In this way the eye patch 10 can be removed from medial or lateral edges of the eye socket 50. As the eye patch 10 is removed in a direction parallel to the edge of the eyelids the chance of the eyelids 21, 22 being pulled apart is reduced and injury is inhibited because the transparent film is gradually removed along the length of the edges of the eyelids.

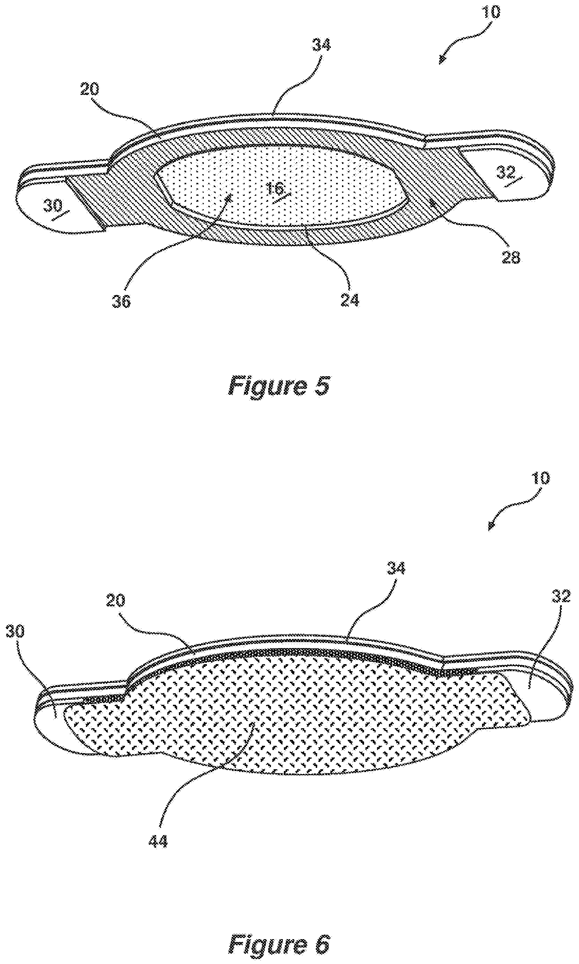

The backing is at least partially covered by a peelable protective sheet 44 prior to use, as illustrated in figure 6.

A method of constructing an eye patch 10 for retaining a patient's eye 12 closed during a medical procedure, not part of the invention, includes the steps of:
1. selecting a backing 20 having a first adhesive coating 28, or applying a first adhesive coating 28 to an underside of a backing 20;
2. selecting a transparent film 34 having a second adhesive coating 36, or applying a second adhesive coating 36 to an underside of a transparent film 34, wherein the second adhesive coating 36 has weaker adhesive bond strength than the first adhesive coating 28;
3. attaching the transparent film 34 over a central opening 24 of the backing 20 to form a transparent window 16 through the eye patch 10 that includes adhesive coating 36;
4. attaching a lower layer 42 to the underside of the backing 20 to form at least one graspable tab 30 having a non-adhesive surface.

As illustrated in figure 7, the eye patch 10 conforms to the contours of the outer surface of the patient face 17 and eye 12. The contours of the patient's eyelids 21, 22 and surrounding skin 48 are determined by the underlying edge of the eye socket or bony orbital rim 50 and eyeball 52 both indicated by broken lines. The first portion 18 of the eye patch 10 is attached to the skin 48 of the patient circumferentially around the edge of the patient's closed eye 12 by way of the first adhesive coating 28. As illustrated, the first portion 18, comprising the backing 20, is attached to the face 17 at a distance from a respective edge 54 of each eyelid 21, 22 and eyelashes 26.

The transparent film 34 may be water/fluid resistant or repellent. This inhibits skin preparations or fluids/oils, used during the medical procedure, from penetrating through the eye patch and coming into contact with the eye. As the skilled addressee would appreciate, even if the eye were kept closed, fluids and other liquids could seep into the eye between the abutting eyelids thereby damaging or irritating the cornea.

The eye patch 10 is therefore configured to inhibit fluids and creams, used during the medical procedure, from penetrating through the eye patch or seeping under the edge of the eye patch and into the eye, which could result in irritation or injury. Accordingly, the eye patch acts to both keep the eye closed and to inhibit chemicals from coming into contact with the edge of the closed eyelids, which may affect the patient.

The configuration of the eye patch 10 and attachment of the first and second portions 18, 19 to different parts of the face 17, thereby retain the eye patch 10 in position during the medical procedure to inhibit the eyelids 21, 22 from opening, while inhibiting injury to the patient's eyelids 21, 22 when the eye patch 10 is removed at the end of the medical procedure.

Figure 8 illustrates a third embodiment of the eye patch wherein the second portion 19, comprising the transparent film 34, is spaced apart from the tabs 30, 32 that are positioned at opposite sides of the backing 20. In this embodiment the transparent film 34 does not extend across the whole of the eye patch 10, rather it is attached circumferentially around the edge of the central opening 24 of backing 20.

The skilled addressee will now appreciate the advantages of the illustrated invention over the prior art. In one form the invention provides an eye patch 10 having a central transparent window 16 through which an anaesthetist can see the eyelids to ensure they are closed throughout the medical procedure. The transparent film 34 has an adhesive coating 36 to inhibit the opening of the eyelids, but has relatively weak bond strength, so that it can be easily removed from the face 17 of the patient without plucking the eyelashes or causing injury to the edges of the patient's eyelids. Patients, especially those that are older, have eyelids that are more susceptible to being torn or bruised by the removal of the medical tape that is often currently used. The backing 20 on the other hand has higher adhesive bond strength to ensure that the eye patch 10 is not easily dislodged during use, due to physical contact or increased moisture on the skin. The illustrated invention provides a convenient means for handling and applying a very light and flexible membrane or film over the eyelids of the patient using a circumferential support.

Various features of the invention have been particularly shown and described in connection with the exemplified embodiments of the invention, however it must be understood that these particular arrangements merely illustrate the invention and it is not limited thereto. Accordingly the invention can include various modifications, which fall within the scope of the invention.

## Claims

1. An eye patch (10) for releasable mounting to a face (17) of a patient (14), used to retain cooperating upper and lower eyelids (21, 22) of the patient closed during a medical procedure, including
a first portion (18) comprising a backing (20) dimensioned to extend circumferentially around the upper and lower eyelids of the patient adjacent an edge of a corresponding eye socket and at a distance from eyelashes of the patient, the backing (20) having a first adhesive coating (28) for attachment to the face (17) of the patient adjacent the edge of the patient's eye socket, the first portion (18) including a central opening (24) extending therethrough, the central opening (24) alignable with a position of respective eyelashes of the upper and lower eyelids (21, 22) when closed, and a second portion (19) comprising a transparent film or membrane (34) extending over the central opening (24), wherein the second portion (19) has a second adhesive coating (36) for attachment to the eyelids (21, 22) of the patient to restrain the eyelids from opening, the second portion (19) extending over the patient's eyelids when closed, such that the eyelids can be observed during a medical procedure,
wherein the adhesive bond strength of the second adhesive coating (36) is weaker than the adhesive bond strength of the first adhesive coating (28), **characterised in that** at least one tab (30, 32) having an outer non-adhesive surface adjoins and extends outwardly from the backing (20), the at least one tab (30, 32) being graspable to remove the eye patch (10) from the medial or lateral edge of the eye socket of the patient (14), and wherein the transparent film or membrane (34) has greater flexibility than the backing, such that the backing (20) provides a degree of rigidity or stiffness whereby the transparent film or membrane (34) extends over the central opening (24) and held by the circumferentially extending backing (20), to assist a person when handling the eye patch and to inhibit injury to the patient's face when the eye patch (10) is removed.

2. The eye patch (10) according to claim 1, wherein the backing (20) has a thickness that is greater than the transparent film or membrane (34), whereby the ratio of thickness of the backing (20) relative to the transparent film or membrane (34) is selected from a range of ratios between 2:1 and 10:1.

3. The eye patch (10) according to claim 2, wherein the transparent film or membrane (34) has a thickness of 0.5mm, and the backing (20) has a thickness of 2mm.

4. The eye patch (10) according to any preceding claim, wherein the rigidity or stiffness of the backing (20) is generated by having the backing formed from a different material compared to the transparent film or membrane (34), or by the backing (20) having ridges, or by the backing (20) having a composite construction.

5. The eye patch (10) according to any preceding claim, wherein the backing (20) further extends over an outer part of the upper and lower eyelids (21, 22) at the distance from an edge of the eyelids and the eyelashes.

6. The eye patch (10) according to any preceding claim, wherein the first and second adhesive coatings (28, 36) are pressure-sensitive hypoallergenic adhesives having different adhesive bond strengths.

7. The eye patch (10) according to any preceding claim, wherein the central opening (24) of the backing (20) and the transparent film or membrane (34) form a transparent viewing window that permits an anaesthetist or other medical practitioner to view the eyelids (21, 22) when the eye patch (10) is attached to the patient to ensure the eyelids are closed throughout the medical procedure.

8. The eye patch (10) according to any preceding claim, wherein the first and second portions (18, 19) are fluid resistant or fluid repellent to inhibit fluids from penetrating inwardly through the eye patch (10) from an exterior surface.

9. The eye patch (10) according to any preceding claim, including two spaced apart non-peelable tabs (30, 32), one at each side of the eye patch, each having a respective non-adhesive outer surface wherein the eye patch (10) can be removed from the medial or lateral edge of the patient's eye socket.

10. The eye patch (10) according to claim 9, wherein the eye patch is constructed from three layers, an upper layer comprising the transparent film (34) having an underside covered by the second adhesive coating (36), a middle layer comprising the backing (20) having the central opening (24) extending therethrough and an underside covered by the first adhesive coating (28), and a lower layer comprising the two spaced apart tabs (30, 32) having non-adhesive underside surfaces, the upper and middle layers having similar outer dimensions.

## Patentansprüche

1. Augenpflaster (10) zum lösbaren Anbringen an einem Gesicht (17) eines Patienten (14), das dafür verwendet wird, zusammenwirkende obere und untere Augenlider (21, 22) des Patienten während eines medizinischen Eingriffs geschlossen zu halten, Folgendes einschließend:
einen ersten Abschnitt (18), der einen Träger (20) umfasst, der so bemessen ist, dass er sich umlaufend um das obere und das untere Augenlid des Patienten angrenzend an einen Rand einer entsprechenden Augenhöhle und in einem Abstand von Augenwimpern des Patienten erstreckt, wobei der Träger (20) eine erste Klebstoffbeschichtung (28) zur Befestigung am Gesicht (17) des Patienten angrenzend an den Rand der Augenhöhle des Patienten aufweist, wobei der erste Abschnitt (18) eine mittige Öffnung (24) einschließt, die sich durch denselben erstreckt, wobei die mittige Öffnung (24) mit einer Position jeweiliger Augenwimpern des oberen und des unteren Augenlids (21, 22) ausgerichtet werden kann, wenn sie geschlossen sind, und einen zweiten Abschnitt (19), der eine transparente Folie oder Membran (34) umfasst, die sich über die mittige Öffnung (24) erstreckt,
wobei der zweite Abschnitt (19) eine zweite Klebstoffbeschichtung (36) zur Befestigung an den Augenlidern (21, 22) des Patienten aufweist, um die Augenlider daran zu hindern, sich zu öffnen, wobei sich der zweite Abschnitt (19) über die Augenlider des Patienten erstreckt, wenn sie geschlossen sind, so dass die Augenlider während eines medizinischen Eingriffs beobachtet werden können, wobei die Klebehaftfestigkeit der zweiten Klebstoffbeschichtung (36) schwächer ist als die Klebehaftfestigkeit der ersten Klebstoffbeschichtung (28),
**dadurch gekennzeichnet, dass**
sich mindestens eine Lasche (30, 32), die eine äußere nicht-klebende Fläche aufweist, an den Träger (20) anschließt und außerhalb desselben erstreckt, wobei die mindestens eine Lasche (30, 32) greifbar ist, um das Augenpflaster (10) von dem medialen oder lateralen Rand der Augenhöhle des Patienten (14) zu entfernen,
und
wobei die transparente Folie oder Membran (34) eine größere Flexibilität aufweist als der Träger, so dass der Träger (20) einen Grad an Starrheit oder Steifigkeit bereitstellt, wodurch sich die transparente Folie oder Membran (34) über die mittige Öffnung (24) erstreckt und durch den sich umlaufend erstreckenden Träger (20) gehalten wird, um eine Person zu unterstützen, wenn sie das Augenpflaster handhabt, und um eine Verletzung des Gesichts des Patienten zu verhindern, wenn das Augenpflaster (10) entfernt wird.

2. Augenpflaster (10) nach Anspruch 1, wobei der Träger (20) eine Dicke aufweist, die größer ist als die transparente Folie oder Membran (34), wodurch das Dickenverhältnis des Trägers (20) relativ zu der transparenten Folie oder Membran (34) aus einem Bereich von Verhältnissen zwischen 2:1 und 10:1 ausgewählt ist.

3. Augenpflaster (10) nach Anspruch 2, wobei die transparente Folie oder Membran (34) eine Dicke von 0,5 mm aufweist und der Träger (20) eine Dicke von 2 mm aufweist.

4. Augenpflaster (10) nach einem der vorhergehenden Ansprüche, wobei die Starrheit oder Steifigkeit des Trägers (20) dadurch erzeugt wird, dass der Träger aus einem unterschiedlichen Material, verglichen mit der transparenten Folie oder Membran (34), hergestellt ist, oder dadurch, dass der Träger (20) Stege aufweist, oder dadurch, dass der Träger (20) einen Verbundaufbau aufweist.

5. Augenpflaster (10) nach einem der vorhergehenden Ansprüche, wobei sich der Träger (20) ferner über einen äußeren Teil des oberen und des unteren Augenlides (21, 22) in dem Abstand von einem Rand der Augenlider und den Augenwimpern erstreckt.

6. Augenpflaster (10) nach einem der vorhergehenden Ansprüche, wobei die erste und die zweite Klebstoffbeschichtung (28, 36) druckempfindliche hypoallergene Klebstoffe sind, die unterschiedliche Klebehaftfestigkeiten aufweisen.

7. Augenpflaster (10) nach einem der vorhergehenden Ansprüche, wobei die mittige Öffnung (24) des Trägers (20) und die transparente Folie oder Membran (34) ein transparentes Sichtfenster bilden, das es einem Anästhesisten oder einem anderen Mediziner ermöglicht, die Augenlider (21, 22) zu sehen, wenn das Augenpflaster (10) an dem Patienten befestigt ist, um sicherzustellen, dass die Augenlider durch den gesamten medizinischen Eingriff geschlossen sind.

8. Augenpflaster (10) nach einem der vorhergehenden Ansprüche, wobei der erste und der zweite Abschnitt (18, 19) fluidbeständig oder fluidabweisend sind, um Fluid daran zu hindern, von einer äußeren Fläche durch das Augenpflaster (10) nach innen einzudringen.

9. Augenpflaster (10) nach einem der vorhergehenden Ansprüche, das zwei voneinander beabstandete nicht-abziehbare Laschen (30, 32), eine auf jeder Seite des Augenpflasters, einschließt, die jede eine jeweilige nicht-klebende Außenfläche aufweisen, wobei das Augenpflaster (10) von dem medialen oder lateralen Rand der Augenhöhle des Patienten entfernt werden kann.

10. Augenpflaster (10) nach Anspruch 9, wobei das Augenpflaster aus drei Lagen aufgebaut ist, einer oberen Lage, welche die transparente Folie (34) umfasst, die eine Unterseite aufweist, die von der zweiten Klebstoffbeschichtung (36) bedeckt ist, eine mittlere Lage, die den Träger (20) umfasst, der die mittige Öffnung (24), die sich durch denselben erstreckt, und eine Unterseite, die von der ersten Klebstoffbeschichtung (28) bedeckt ist, aufweist, und eine untere Lage, welche die zwei voneinander beabstandeten Laschen (30, 32) umfasst, die nicht-klebende Unterseitenflächen aufweisen, wobei die obere und die mittlere Lage ähnliche äußere Abmessungen aufweisen.

## Revendications

1. Pansement oculaire (10) pour une application de manière amovible sur un visage (17) d'un patient (14), utilisé pour retenir les paupières supérieure et inférieure coopérantes (21, 22) du patient fermées au cours d'une procédure médicale, incluant :
une première partie (18) comprenant un support (20) dimensionné de sorte à s'étendre circonférentiellement autour des paupières supérieure et inférieure du patient près d'un bord d'une orbite correspondante et à distance des cils du patient, le support (20) comportant un premier revêtement adhésif (28) destiné à être attaché sur le visage (17) du patient près du bord de l'orbite du patient, la première partie (18) incluant une ouverture centrale (24) la traversant, l'ouverture centrale (24) pouvant être alignée avec une position des cils respectifs des paupière supérieure et inférieure (21, 22) lorsqu'elles sont fermées, et une deuxième partie (19) comprenant un film transparent ou une membrane (34) s'étendant au-dessus de l'ouverture centrale (24),
dans lequel la deuxième partie (19) comporte un deuxième revêtement adhésif (36) destiné à être appliqué sur les paupières (21, 22) du patient pour empêcher les paupières de s'ouvrir, la deuxième partie (19) s'étendant au-dessus des paupières du patient lorsqu'elles sont fermées, de sorte que les paupières peuvent être observées au cours d'une procédure médicale, dans lequel la résistance d'adhésion du deuxième revêtement adhésif (36) est plus faible que la résistance d'adhésion du premier revêtement adhésif (28),
**caractérisé en ce que** :
au moins une languette (30, 32) comportant une surface externe non adhésive est adjacente au support (20) et s'étend vers l'extérieur de celui-ci, la au moins une languette (30, 32) pouvant être saisie pour retirer le pansement oculaire (10) du bord médial ou latéral de l'orbite du patient (14) ;
et
dans lequel le film transparent ou la membrane (34) présente une flexibilité supérieure à celle du support, de sorte que le support (20) établit un degré de rigidité ou de raideur, le film transparent ou la membrane (34) s'étendant ainsi au-dessus de l'ouverture centrale (24) et étant retenu par le support à extension circonférentielle (20), pour assister une personne lors de la manipulation du pansement oculaire et pour empêcher une blessure du visage du patient lors du retrait du pansement oculaire (10).

2. Pansement oculaire (10) selon la revendication 1, dans lequel le support (20) a une épaisseur supérieure à celle du film transparent ou de la membrane (34), le rapport entre l'épaisseur du support (20) et celle du film transparent ou de la membrane (34) étant ainsi sélectionnée dans un intervalle de rapport compris entre 2 : 1 et 10 : 1.

3. Pansement oculaire (10) selon la revendication 2, dans lequel le film transparent ou la membrane (34) a une épaisseur de 0,5 mm et le support (20) a une épaisseur de 2 mm.

4. Pansement oculaire (10) selon l'une quelconque des revendications précédentes, dans lequel la rigidité ou la raideur du support (20) est générée par la formation du support à partir d'un matériau différent de celui du film transparent ou de la membrane (34) ou par la formation de nervures sur le support ou par une construction composite du support (20).

5. Pansement oculaire (10) selon l'une quelconque des revendications précédentes, dans lequel le support (20) s'étend en outre au-dessus d'une partie externe des paupières supérieure et inférieure (21, 22) à distance d'un bord des paupières et des cils.

6. Pansement oculaire (10) selon l'une quelconque des revendications précédentes, dans lequel les premier et deuxième revêtements adhésifs (28, 36) sont des adhésifs hypoallergéniques sensibles à la pression présentant différentes résistances d'adhésion.

7. Pansement oculaire (10) selon l'une quelconque des revendications précédentes, dans lequel l'ouverture centrale (24) du support (20) et le film transparent et ou la membrane (34) forment une fenêtre de visualisation transparente permettant à un anesthésiste ou à un autre médecin qualifié de voir les paupières (21, 22) lorsque le pansement oculaire (10) est fixé sur le patient, pour qu'il s'assure que les paupières sont fermées pendant l'ensemble de la procédure médicale.

8. Pansement oculaire (10) selon l'une quelconque des revendications précédentes, dans lequel les première et deuxième parties (18, 19) sont résistantes au fluide ou répulsives contre le fluide pour empêcher la pénétration de fluides vers l'intérieur à travers le pansement oculaire (10) à partir d'une surface externe.

9. Pansement oculaire (10) selon l'une quelconque des revendications précédentes, incluant deux languettes non détachables espacées (30,32), l'une au niveau de chaque côté du pansement oculaire, comportant chacune une surface externe non adhésive respective, dans lequel le pansement oculaire (20) peut être retiré du bord médial ou latéral de l'orbite de l'œil du patient.

10. Pansement oculaire (10) selon la revendication 9, dans lequel le pansement oculaire est composé de trois couches, d'une couche supérieure comprenant le film transparent (34) comportant un côté inférieur recouvert par le deuxième revêtement adhésif (36), une couche médiane comprenant le support (20) comportant l'ouverture centrale (24) le traversant et un côté inférieur recouvert par le premier revêtement adhésif (28), et une couche inférieure comprenant les deux languettes espacées (30,32) comportant des surfaces inférieures non adhésives, les couches supérieure et médiane ayant des dimensions externes similaires.
